(19) Europäisches Patentamt European Patent Office Office européen des brevets

(11) **EP 4 600 903 B1**

(12) **EUROPEAN PATENT SPECIFICATION**

(45) Date of publication and mention of the grant of the patent:
**29.07.2026 Bulletin 2026/31**

(21) Application number: **25156159.3**

(22) Date of filing: **06.02.2025**

(51) International Patent Classification (IPC):
***G06T 7/50*** *(2017.01)*

(52) Cooperative Patent Classification (CPC):
**G06T 7/50; A61F 2/30942; A61F 2/46; G06N 3/02; G06N 3/08; G06N 20/00;** A61B 2034/104; A61B 2034/105; A61F 2/3872; A61F 2002/30948; A61F 2002/4633; G06T 2207/10088; G06T 2207/10116; G06T 2207/20084; G06T 2207/30008

(54) **DEVICE AND METHOD FOR PREDICTING TERTIARY STRUCTURE OF PATIENT-TAILORED IMPLANTS**

VORRICHTUNG UND VERFAHREN ZUR VORHERSAGE DER TERTIÄRSTRUKTUR VON AUF PATIENTEN ZUGESCHNITTENEN IMPLANTATEN

DISPOSITIF ET PROCÉDÉ DE PRÉDICTION DE STRUCTURE TERTIAIRE D'IMPLANTS ADAPTÉS À UN PATIENT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **06.02.2024 KR 20240018220**

(43) Date of publication of application:
**13.08.2025 Bulletin 2025/33**

(73) Proprietors:
• **Seoul Women's University Industry-University Cooperation Foundation**
  **Seoul 01797 (KR)**
• **Samsung Life Public Welfare Foundation**
  **Seoul 04348 (KR)**

(72) Inventors:
• **HONG, Helen Kim**
  **06602 Seocho-gu, Seoul (KR)**
• **KIM, Hyeonjin**
  **04941 Gwangjin-gu, Seoul (KR)**
• **WANG, Joon Ho**
  **06351 Gangnam-gu, Seoul (KR)**

(74) Representative: **dompatent Partnerschaft von Patentanwälten und Rechtsanwälten mbB Deichmannhaus am Dom Bahnhofsvorplatz 1 50667 Köln (DE)**

(56) References cited:
**KR-B1- 102 228 087    US-B2- 8 965 088**

• **VRANCKEN A. C. ET AL: "3D geometry analysis of the medial meniscus - a statistical shape modeling approach", JOURNAL OF ANATOMY., vol. 225, no. 4, 23 July 2014 (2014-07-23), GB, pages 395 - 402, XP093287988, ISSN: 0021-8782, DOI: 10.1111/joa.12223**
• **LEWIS KIRA ET AL: "Calculation of the Meniscus Shape Formed under Gravitational Force by Solving the Young-Laplace Differential Equation Using the Bézier Curve Method", ACS OMEGA, vol. 7, no. 41, 7 October 2022 (2022-10-07), US, pages 36510 - 36518, XP093287994, ISSN: 2470-1343, DOI: 10.1021/acsomega.2c04359**
• **TACK A. ET AL: "Knee menisci segmentation using convolutional neural networks: data from the Osteoarthritis Initiative", OSTEOARTHRITIS AND CARTILAGE, vol. 26, no. 5, May 2018 (2018-05-01), AMSTERDAM, NL, pages 680 - 688, XP093288057, ISSN: 1063-4584, DOI: 10.1016/ j.joca.2018.02.907**

EP 4 600 903 B1

**Description**

[TECHNICAL FIELD]

Embodiments of the present disclosure described herein relate to an apparatus and a method for predicting a 3D shape of a patient-tailored implant, and, more particularly, relate to an apparatus and a method for predicting a 3D shape of a damaged knee cartilage to fabricate a patient-tailored implant structure.

[BACKGROUND ART]

Unless otherwise stated in the present disclosure, the contents described in this section do not constitute prior art to the claims of this application, nor should they be regarded as prior art merely by being included in this section.
Meniscus transplantation is one of the surgical operations performed on patients with damaged joint or cartilage. The meniscus transplantation is primarily used in the knee joint to repair damage to the meniscus, the C-shaped cartilage that sits between the kneecap and femur in the knee joint. The meniscus transplantation must be performed to replace the meniscus before the cartilage joint wears out, and the exact size of the cartilage is the most critical factor for the success of the procedure. However, there is no existing method for predicting the tertiary structure of a patient-tailored implant.
Related prior art is known from US8965088B2, KR102228087B1, Vrancken A. C. et al.: "3D geometry analysis of the medial meniscus - a statistical shape modeling approach", Lewis Kira et al.: "Calculation of the Meniscus Shape Formed under Gravitational Force by Solving the Young-Laplace Differential Equation Using the Bézier Curve Method", and A. Tack et al.: "Knee menisci segmentation using convolutional neural networks: data from the Osteoarthritis Initiative".

[DETAILED DESCRIPTION OF THE INVENTION]

[TECHNICAL PROBLEM]

Embodiments of the present disclosure provide an apparatus and a method for predicting a 3D shape of a patient-tailored implant, which enable the fabrication of a structure of a patient-tailored implant by predicting the 3D shape of a damaged knee cartilage (meniscus).

[TECHNICAL SOLUTION]

The invention is defined in the appended claims.
According to an embodiment, an apparatus for predicting a 3D shape of a patient-tailored implant includes a memory that stores at least one instruction for predicting the 3D shape of the patient-tailored implant and a processor that executes an operation according to the instruction, wherein the processor models a correlation between a 2D meniscus shape and a 3D meniscus shape, and predicts the 3D meniscus shape based on 3D shape features derived from modeling results.
According to an embodiment, a method for predicting a 3D shape of a patient-tailored implant, the method being performed by a processor of an apparatus includes modeling a correlation between a 2D meniscus shape and a 3D meniscus shape, and predicting the 3D meniscus shape based on 3D shape features derived from modeling results.

[DESCRIPTION OF THE DRAWINGS]

FIG. 1 is a diagram showing a CT image, a 3D modeling image, and a predicted 3D meniscus obtained through a prediction model which are used in the present disclosure.
FIG. 2 is a diagram for describing a data processing method for the apparatus for predicting the 3D shape of a patient-tailored implant according to the present disclosure.
FIG. 3 is a block diagram of the apparatus for predicting the 3D shape of a patient-tailored implant according to the present disclosure.
FIG. 4 is a diagram for describing stress X-ray images used in the present disclosure.
FIG. 5 is a diagram showing a meniscus shape prediction model according to the present disclosure.
FIG. 6 is a diagram illustrating an example of performing K-means clustering according to the present disclosure.
FIG. 7 is a diagram showing a process of predicting the 3D shape of a patient-tailored implant according to the present disclosure.

[BEST MODE]

Hereinafter, the present disclosure will be described in detail with reference to the accompanying drawings, and the same or similar elements are designated with the same numeral references regardless of the numerals in the drawings and their redundant description will be omitted. The suffixes "module" and "unit or portion" for components used in the following description are merely provided only for facilitation of preparing this specification, and thus they are not granted a specific meaning or function. In addition, when it is determined that the detailed description of the related known technology may obscure the gist of embodiments disclosed herein in describing the embodiments, a detailed description thereof will be omitted. Further, the accompanying drawings are intended to facilitate understanding of the embodiments disclosed herein, and the technical spirit disclosed herein are not limited by the accompanying drawings. Therefore, the present disclosure should be construed as including all the changes, equivalents, and substitutions included in the spirit and scope of the present disclosure.

The terms coming with ordinal numbers such as 'first', 'second', or the like may be used to denote various components, but the components are not limited by the terms. The terms are used merely for the purpose to distinguish a component from the other component.

It will also be understood that when an element is referred to as being "connected" or "coupled" to another element, it may be directly connected or coupled to the other element or intervening elements may be present. In contrast, when an element is referred to as being "directly connected" or "directly coupled" to another element, there are no intervening elements present.

It will be further understood that the terms "comprises," "comprising," "having," "having," "includes," "including" and/or variations thereof, when used in this specification, specify the presence of stated features, integers, steps, operations, elements, and/or components, but do not preclude the presence or addition of one or more other features, integers, steps, operations, elements, components, and/or groups thereof.

In the present specification, the term "unit" includes a unit implemented by hardware, a unit implemented by software, and a unit implemented by both the hardware and software. As examples only, one unit may be implemented by two or more pieces of hardware or two or more units may be implemented by one piece of hardware.

Some of the operations or functions described herein as being performed by a terminal, apparatus, or device may instead be performed by a server associated with the terminal, apparatus, or device. Similarly, some of the operations or functions described as being performed by a server may also be performed by a terminal, apparatus, or device associated with the server.

Hereinafter, the present disclosure will be described in detail with reference to the accompanying drawings.

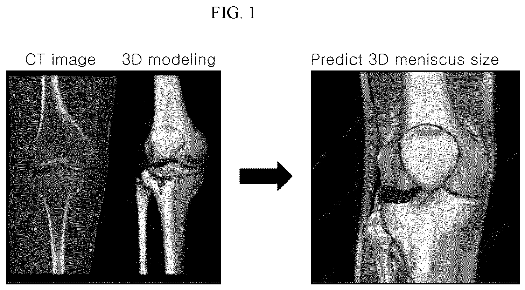

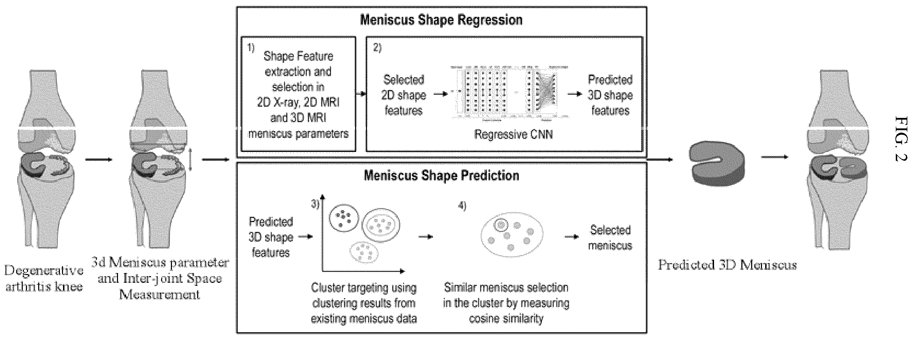

FIG. 1 is a diagram illustrating a CT image and a 3D modeling image, and a 3D meniscus predicted through a prediction model which are used in the present disclosure. FIG. 2 is a diagram for describing a data processing method for an apparatus for predicting a 3D shape of patient-tailored implant according to the present disclosure.

The apparatus for predicting a 3D shape of patient-tailored implant 100 according to the present disclosure may measure 3D meniscus parameters and the joint space and performs meniscus shape regression to model the correlation between the 2D meniscus shape and the 3D meniscus shape. Subsequently, the apparatus for predicting a 3D shape of patient-tailored implant 100 may predict the shape of the 3D meniscus based on the 3D shape feature derived from the modeling results. Thereafter, the present disclosure may select a meniscus most similar to the predicted 3D meniscus.

The apparatus for predicting a 3D shape of patient-tailored implant and a method according to the present disclosure may predict the 3D shape of a damaged knee cartilage (meniscus) to enable the fabrication of a patient-tailored implant shape.

The apparatus for predicting a 3D shape of patient-tailored implant and a method according to the present disclosure may measure the 2D meniscus shape features from the 2D X-ray and 2D MRI images of the opposite, healthy knee of an identical patient and, through correlation analysis, select shape features with a high correlation between the 2D shape features and the 3D shape features.

Subsequently, the selected 2D features and demographic features may be input into a prediction model that includes a regressive CNN.

Thereafter, the prediction model may predict values for the shape features of the 3D meniscus and generate a meniscus group to find out similar meniscus among a plurality of normal human meniscus data stored in advance (pre-stored).

Then, after finding out and selecting a cluster with the most similar mean value and standard deviation of the shape of the predicted 3D meniscus to the cluster by the above generated meniscus group, similar features may be extracted through cosine similarity between the shape features of the predicted 3D meniscus and the shape features of training data in the selected cluster, and the most similar shape model of a normal person may be obtained based on the extracted similar features to select a similar meniscus.

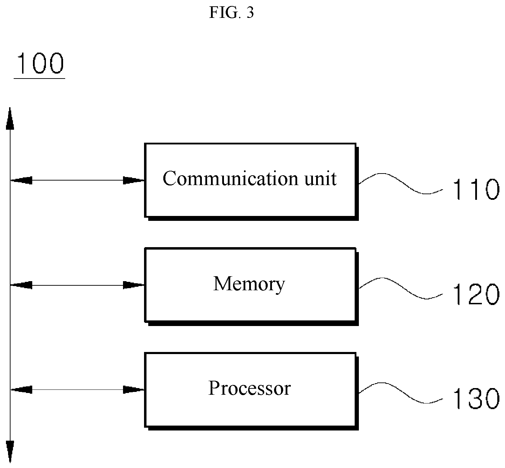

FIG. 3 is a block diagram of the apparatus for predicting a 3D shape of patient-tailored implant according to the present disclosure.

In the present disclosure, the apparatus for predicting a 3D shape of patient-tailored implant 100 may include a server, which is a computing system that provides services to other computers or devices within a computer network or stores and manages data. The apparatus for predicting a 3D shape of patient-tailored implant 100 may receive a request from other computers or devices, called clients, and provide a response or data for the request. The configuration of the apparatus for predicting a 3D shape of patient-tailored implant 100 shown in FIG. 3 is a simplified example.

A communication unit 110 may be configured in various communication methods, including wired and wireless communication methods, and may perform communication via diverse communication networks such as a Personal Area Network (PAN) or a Wide Area Network (WAN). Further, the communication unit 110 may operate based on the well-known World Wide Web (WWW) and may use wireless transmission technologies used in short-range communication such as Infrared Data Association (IrDA) or Bluetooth™. For example, the communication unit 110 may be responsible for transmitting and receiving data necessary for performing the techniques of the present disclosure.

A memory 120 may represent any type of storage medium. For example, the memory 120 may include at least one type of storage medium among a flash memory type, a hard disk type, a multimedia card micro type, a card type memory (for example, SD or XD memory), RAM (Random Access Memory), SRAM (Static Random Access Memory), ROM (Read-Only Memory), EEPROM (Electrically Erasable Programmable Read-Only Memory), PROM (Programmable Read-Only Memory), a magnetic memory, a magnetic disk, and an optical disk. The memory 120 may also configure the database shown in FIG. 1.

The memory 120 may store at least one instruction executable by a processor 130. Additionally, the memory 120 may store any form of information generated or determined by the processor 130 and any form of information received by a server 200. For example, the memory 120 may store RM data and RM protocols for users, as will be described later. Moreover, the memory 120 may store various types of modules, instruction sets, and models.

In the present disclosure, the processor 130 may measure the shape features of 2D meniscus and 3D meniscus from 2D X-ray, 2D MRI, and 3D MRI images of an identical patient's knee to determine the correlation between the 2D meniscus and the 3D meniscus.

In the present disclosure, the processor 130 may measure the 2D meniscus shape features from 2D X-ray and 2D MRI images, performs automatic meniscus segmentation on 3D MRI images, and subsequently measure the 3D meniscus shape features based on the results of the automatic meniscus segmentation.

The processor 130 may then perform a correlation analysis to select 2D shape features that have a correlation between the 2D shape feature and the 3D shape feature above a predetermined level.

In the present disclosure, the 2D shape features may include at least one or more of the following: anteroposterior (AP) length, mediolateral (ML) length, anterior horn-posterior horn (AH-PH) distance, medial femoral condylar (Med FC) length, lateral femoral condylar (Lat FC) length, and joint space in stress X-ray.

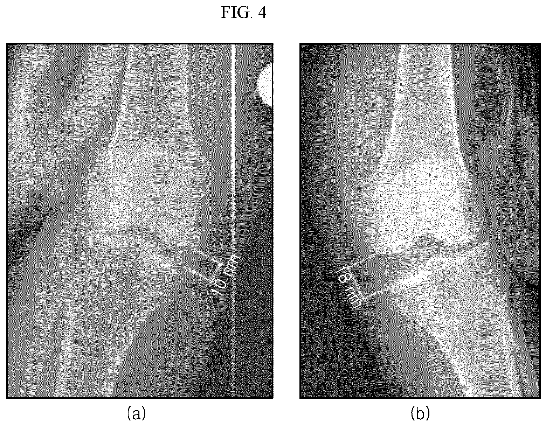

FIG. 4 illustrates a diagram for describing stress X-ray images used in the present disclosure. FIG. 4A shows a standard X-ray, and FIG. 4B shows a stress X-ray, where the knee is pressed sideways to create space within the joints.

The selected demographic feature in the present disclosure includes height. Meanwhile, the 3D space features may include at least one or more of the following: AP length, ML length, AH-PH distance, inner circumference, outer circumference, coverage area, and gap area. Further, as used herein, the demographic feature is a specific feature selected from among statistical and descriptive features of a given population or group, wherein the demographic feature provides information about a specific subset of the population.

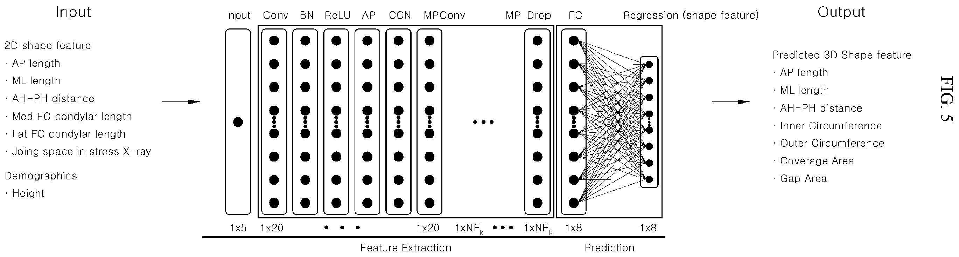

FIG. 5 illustrates the meniscus shape prediction model according to the present disclosure.

Referring to FIG. 5, the processor 130 may perform meniscus shape regression using the meniscus shape prediction model.

The meniscus shape regression refers to a process of estimating parameters of a relevant model from given data in statistics and machine learning. The meniscus shape prediction model in the present disclosure may be a model of predicting or estimating the shape of a meniscus positioned in the joint, particularly the two crescent-shaped pieces of cartilage on the sides of the knee. In the present disclosure, the meniscus shape prediction model may include a regression CNN (Convolutional Neural Network).

As shown in FIG. 5, the meniscus shape prediction model may perform regressive CNN by using, as inputs, 2D shape features and demographic features from among the selected features, and predict the values of 3D meniscus shape features.

The input of the meniscus shape prediction model may be 2D shape features and demographic features, where the 2D shape features may include at least one or more of AP length, ML length, AH-PH distance, Med FC condylar length, Lat FC condylar length, and joint space in stress X-ray. The demographic feature includes height.

The output of the meniscus shape prediction model may be predicted 3D shape features, which may include at least one or more of AP length, ML length, AH-PH distance, inner circumference, outer circumference, coverage area, and gap area.

Subsequently, the processor 130 may perform cluster targeting. In the present disclosure, the processor 130 may generate a meniscus group to find out a similar meniscus from pre-stored meniscus data, thereby reducing the time required for selecting a similar meniscus.

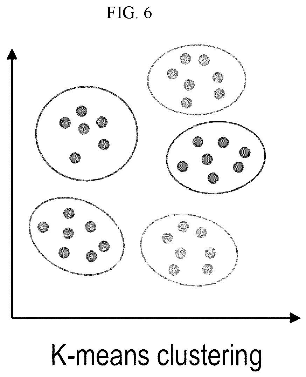

FIG. 6 illustrates an example of performing K-means clustering according to the present disclosure. The processor 130 may perform K-means clustering into K groups using measured 3D shape parameters. The processor 130 may calculate the mean of 3D shape parameters in each cluster.

Then, the processor 130 may measure cosine similarity between the predicted 3D meniscus shape features and the mean features of each cluster to target the most similar cluster.

Afterward, the processor 130 may select a similar meniscus. That is, the processor 130 may measure the cosine similarity between the predicted 3D meniscus shape features and the shape features of the training data within the selected cluster to detect and select the most similar meniscus.

In the present disclosure, the processor 130 may calculate the cosine similarity using the feature vector of the predicted 3D meniscus and the feature vector of the mean cluster. In the present disclosure, the cosine similarity may be calculated using Equation 1.

[Equation 1]

$$\text{similarity} = \cos(\theta) = \frac{A \cdot B}{\|A\|\|B\|} = \frac{\sum_{i=1}^{n} A_i \times B_i}{\sqrt{\sum_{i=1}^{n} (A_i)^2} \times \sqrt{\sum_{i=1}^{n} (B_i)^2}}$$

In Equation 1, $A_i$ is the i-th feature vector of the predicted 3D meniscus, and $B_i$ is the feature vector of the mean cluster or the feature vector of the training data.

In the following, a method for predicting a 3D shape of patient-tailored implant will be described. Since the action (function) of the method for predicting a 3D shape of patient-tailored implant according to the present disclosure is essentially the same as the function of the apparatus for predicting a 3D shape of patient-tailored implant, the description that overlaps with FIGS. 1 to 6 will be omitted.

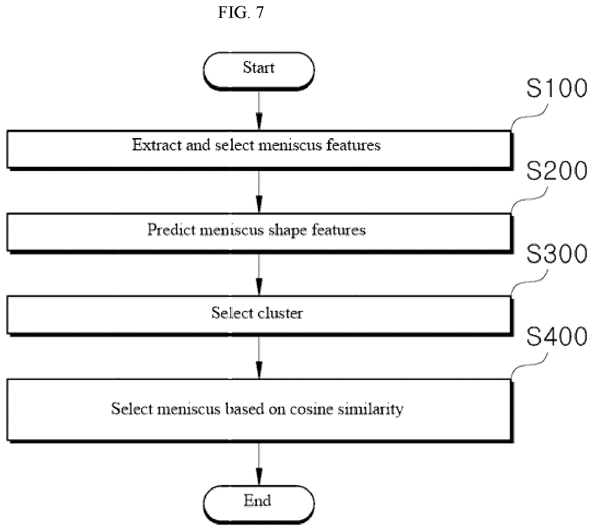

FIG. 7 is a diagram illustrating a process of predicting a 3D shape of a patient-tailored implant according to the present disclosure.

Referring to FIG. 7, in step S100, 2D meniscus shape features may be measured from the 2D X-ray and 2D MRI images of the opposite, healthy knee of an identical patient and shape features to determine the correlation between the 2D meniscus and the 3D meniscus and shape features with high correlation between the 2D feature features and the 3D features may be selected through correlation analysis.

Then, in step S200, regressive CNN may be performed by using. as inputs, the selected 2D features and demographic features and then the shape feature values of the 3D meniscus may be predicted.

In step S300, a meniscus group is generated to find out a similar meniscus from a plurality of normal human meniscus databases.

The generation of the meniscus group may be performed by clustering the mean and variation of the 3D shapes of normal human menisci. Then, a cluster with the most similar mean values and standard deviation values to the predicted shape among the clusters may be detected and selected.

In step S400, a similar meniscus may be selected. That is, similar features may be extracted through cosine similarity between the shape features of the predicted 3D meniscus and the shape features of training data in the selected cluster, and the most similar shape model of a normal person may be obtained among the extracted similar features to select a similar meniscus.

A model according to the present disclosure may refer to any type of computer program that operates based on a network function, an artificial neural network, and/or a neural network. Throughout the present disclosure, the model, the neural network, the network function, and the neural network may be used interchangeably. In the neural network, one or more nodes are interconnected through one or more links to form input and output node relationships within the neural network. The characteristics of the neural network may be determined according to the number of nodes and links within the neural network, the relationship between the nodes and links, and the value of a weight assigned to each link. The neural network may consist of a set of one or more nodes. A subset of nodes constituting the neural network may constitute a layer.

A deep neural network (DNN) may refer to a neural network that contains multiple hidden layers in addition to an input layer and an output layer. Deep neural networks include convolutional neural networks (CNNs), recurrent neural networks (RNNs), auto encoders, generative adversarial networks (GANs), restricted boltzmann machines (RBM), deep belief network (DBN), Q network, U network, Siamese network, generative adversarial network (GAN), transformer, and the like. The foregoing description of deep neural networks is by way of example only and is not intended to limit the present disclosure.

The neural network may be trained in at least one of supervised learning, unsupervised learning, semi-supervised learning, self-supervised learning, or reinforcement learning. Training of the neural network may be a process of applying knowledge for the neural network to perform a specific operation to the neural network.

The neural network may be trained to minimize output errors. When the neural network is trained, training data is repeatedly input into the neural network, the output of the neural network and the error of the target for the training data are calculated, and the error of the neural network is back propagated from the output layer of the neural network to the input layer in the direction of reducing the error to update the weight of each node of the neural network. Supervised learning uses labeled data where each training data is labeled with a correct answer. In contrast, unsupervised learning uses unlabeled data where each training data is not labeled with a correct answer. The amount of change in the connection weights of each node which are updated may be determined by the learning rate. The computation of the neural network on the input data and the backpropagation of errors may constitute a learning cycle (epoch). The learning rate may vary depending on the number of iterations of the learning cycle of the neural network. Furthermore, to prevent overfitting, methods such as increasing training data, regularization, dropout to disable some of the nodes, batch normalization layer, etc. may be applied.

In the present disclosure, the model may adopt at least part of a transformer. The transformer may include an encoder encoding embedded data and a decoder decoding encoded data. The transformer may have a structure that receives a series of data and outputs a series of data of different types through encoding and decoding operations. In the present disclosure, the series of data may be processed in a form computable by the transformer. The process of processing the series of data to the form computable by the transformer may include an embedding process. Expressions, such as data token, embedding vector, and embedding token, may refer to data embedded in the form that is processable by the transformer.

In order for the transformer to encode and decode the series of data, the encoder and decoder within the transformer may be processed by utilizing an attention algorithm. The attention algorithm may refer to an algorithm that calculates similarity for one or more keys for a given query, reflects the calculated similarity to a value corresponding to each key, and then calculates an attention value by calculating a weighted sum of values to which the similarity is reflected.

Various types of attention algorithms may be classified according to how set a query, a key, and a value. For example, when the attention is calculated by setting all of the query, the key, and the value all the same, this may mean a self-attention algorithm. When the attention is calculated by reducing a dimension of the embedding vector and calculating an individual attention head for each divided embedding vector in order to process the input series of data in parallel, this may mean a multi-head attention algorithm.

In the present disclosure, the transformer may include a plurality of modules performing the multi-head self-attention algorithm, or the multi-head encoderdecoder algorithm. In the present disclosure, the transformer may also include additional constituent elements, such as an embedding layer, a normalization layer, and a softmax layer, not in the attention algorithm. The method of configuring the transformer by using the attention algorithm may include the method disclosed in Attention Is All You Need, 2017 NIPS, Vaswani et al.

The transformer may be applied to various data domains, such as an embedded natural language, the divided image data, and an audio waveform to convert a series of input data to a series of output data. In order to convert data having various data domains to a series of data inputtable to the transformer, the transformer may generate an embedding vector for the data. The transformer may process additional data expressing a relative locational relationship or phase relationship between the series of input data. Alternatively, the vectors expressing the relative locational relationship or phase relationship between the input data are additionally reflected to the series of input data, so that the series of input data may be embedded. In an example, the relative locational relationship between the series of input data may include a word order within a natural language sentence, a relative locational relationship of each divided image, a time order of the divided audio waveforms, and the like, but the present disclosure is not limited thereto. The process of adding information expressing the relative locational relationship or phase relationship between the series of input data may be referred to as positional encoding.

In the present disclosure, the model may include, but is not limited to, at least one of a recurrent neural network (RNN), a long short term memory (LSTM) network, a deep neural network (DNN), a convolutional neural network (CNN), and a bidirectional recurrent deep neural network (BRDNN).

In the present disclosure, the model may be a model trained by transfer learning. Here, the transfer learning may refer to a learning method in which a pre-trained model with a first task is obtained by being previously trained with a large amount of unlabeled training data in a semi-supervised or self-learning manner, and the pre-trained model is fine-tuned to fit a second task and is trained with the labeled training data in a supervised manner to implement a targeted model.

Through the apparatus and method for predicting the 3D shape of a patient-tailored implant according to the present disclosure, a treatment plan may be developed while considering the individual characteristics of a patient. This optimizes treatment effectiveness and minimizes side effects.

In addition, the present disclosure may help predict and diagnose a patient's condition at an early stage and provide an individualized risk assessment, taking into account the risk factors of a particular patient. This may also help to take preventive measures.

Furthermore, the present disclosure allows for more rapid detection of the most accurate meniscus through clustering.

On the other hand, the disclosed embodiments may be implemented in the form of a recording medium storing instructions executable by a computer. The instructions may be stored in the form of program codes, which, when executed by the processor, may generate program modules to perform the operations of the disclosed embodiments. The recording medium may be implemented as a computer-readable recording medium.

The computer-readable recording medium includes all kinds of recording media in which instructions readable by a computer is stored. For example, the computer-readable recording medium may include read only memory (ROM), random access memory (RAM), magnetic tape, magnetic disc, flash memory, optical data storage, and the like.

The embodiments of the disclosure have been described above with reference to the accompanying drawings. A person skilled in the art to which the present disclosure pertains will understand that the present disclosure may be practiced in forms different from the disclosed embodiments without changing the technical idea or essential features of the present disclosure. The disclosed embodiments are illustrative and should not be construed as limiting.

While the present disclosure has been described with reference to embodiments, it will be apparent to those skilled in the art that various changes and modifications may be made without departing from the spirit and scope of the present disclosure. Therefore, it should be understood that the above embodiments are not limiting, but illustrative.

**Claims**

1. An apparatus for predicting a 3D shape of a patient-tailored implant (100), comprising:

   a memory (120) configured to store at least one instruction for predicting the 3D shape of the patient-tailored implant (100); and
   a processor (130) configured to execute an operation according to the instruction,
   wherein the processor (130) is configured to:

   model a correlation between a 2D meniscus shape and a 3D meniscus shape;
   predict the 3D meniscus shape based on 3D shape features derived from modeling results;
   measure 2D meniscus shape features from 2D X-ray and 2D MRI images of an opposite, healthy knee of an identical patient to determine the correlation between the 2D meniscus shape and the 3D meniscus shape;
   select 2D shape features that have a correlation between the measured 2D shape features and the 3D shape features above a predetermined level by performing a correlation analysis;
   predict values for the 3D meniscus shape features by inputting the selected 2D shape features and demographic features into a meniscus shape prediction model, which includes a regression CNN; and
   generate a meniscus group.

2. The apparatus of claim 1, wherein the processor (130) is configured to:

   perform grouping by performing clustering based on a mean and variation of 3D shapes of normal human menisci to find out a similar meniscus from pre-stored meniscus data; and
   detect and select a cluster that is most similar to the predicted 3D meniscus shape in terms of a mean and standard deviation of the predicted 3D meniscus shape among clusters generated by the clustering.

3. The apparatus of claim 2, wherein the processor (130) is configured to:

   extract similar features using cosine similarity between the predicted 3D meniscus shape features and shape features of training data within the selected cluster; and
   select a most similar meniscus by obtaining a most similar normal human meniscus based on the extracted similar features.

4. The apparatus of claim 1, wherein the 2D shape features include at least one or more of anteroposterior (AP) length, mediolateral (ML) length, anterior horn-posterior horn (AH-PH) distance, medial femoral condylar (Med FC) length, lateral femoral condylar (Lat FC) length, and joint space in stress X-ray.

5. The apparatus of claim 1, wherein the 3D shape features include at least one or more of anteroposterior (AP) length, mediolateral (ML) length, anterior horn-posterior horn (AH-PH) distance, inner circumference, outer circumference, coverage area, and gap area.

6. The apparatus of claim 2, wherein the processor (130) is configured to:

   perform K-means clustering into K groups using measured 3D shape parameters; and
   measure a mean between 3D shape parameters within each cluster.

7. The apparatus of claim 3, wherein the processor (130) is configured to calculate the cosine similarity using a feature vector of the predicted 3D meniscus and a feature vector of a mean cluster.

8. The apparatus of claim 7, wherein the cosine similarity is calculated through the following equation 1, where Ai is an i-th feature vector of the predicted 3D meniscus, and Bi is the feature vector of the mean cluster.

[Equation 1]

$$\text{similarity} = \cos(\theta) = \frac{A \cdot B}{\|A\|\|B\|} = \frac{\sum\limits_{i=1}^{n} A_i \times B_i}{\sqrt{\sum\limits_{i=1}^{n} (A_i)^2} \times \sqrt{\sum\limits_{i=1}^{n} (B_i)^2}}$$

9. A method for predicting a 3D shape of a patient-tailored implant (100), the method being performed by a processor (130) of an apparatus, comprising:

   modeling a correlation between a 2D meniscus shape and a 3D meniscus shape;
   predicting the 3D meniscus shape based on 3D shape features derived from modeling results;
   measuring 2D meniscus shape features from 2D X-ray and 2D MRI images of an opposite, healthy knee of an identical patient to determine the correlation between the 2D meniscus shape and the 3D meniscus shape;
   selecting 2D shape features that have a correlation between the measured 2D shape features and the 3D shape features above a predetermined level by performing a correlation analysis;
   predicting values for the 3D meniscus shape features by inputting the selected 2D shape features and demographic features into a meniscus shape prediction model, which includes a regression CNN; and
   generating a meniscus group.

10. The method of claim 9, further comprising:

    performing grouping by performing clustering based on a mean and variation of 3D shapes of normal human menisci to find out a similar meniscus from pre-stored meniscus data; and
    detecting and selecting a cluster that is most similar to the predicted 3D meniscus shape in terms of a mean and standard deviation of the predicted 3D meniscus shape among clusters generated by the clustering.

11. The method of claim 10, further comprising:

    extracting similar features using cosine similarity between the predicted 3D meniscus shape features and shape features of training data within the selected cluster; and
    selecting a most similar meniscus by obtaining a most similar normal human meniscus based on the extracted similar features.

12. The method of claim 9, wherein the 2D shape features include at least one or more of anteroposterior (AP) length, mediolateral (ML) length, anterior horn-posterior horn (AH-PH) distance, medial femoral condylar (Med FC) length, lateral femoral condylar (Lat FC) length, and joint space in stress X-ray.

13. The method of claim 9, wherein the 3D shape features include at least one or more of anteroposterior (AP) length, mediolateral (ML) length, anterior horn-posterior horn (AH-PH) distance, inner circumference, outer circumference, coverage area, and gap area.

14. The method of claim 10, further comprising:

  performing K-means clustering into K groups using measured 3D shape parameters; and
  measuring a mean between 3D shape parameters within each cluster.

15. The method of claim 11, further comprising:
calculating the cosine similarity using a feature vector of the predicted 3D meniscus and a feature vector of a mean cluster.

16. The method of claim 15, wherein the cosine similarity is calculated through the following equation 1, where Ai is an i-th feature vector of the predicted 3D meniscus, and Bi is the feature vector of the mean cluster.

[Equation 1]

$$\text{similarity} = \cos(\theta) = \frac{A \cdot B}{\|A\|\|B\|} = \frac{\sum_{i=1}^{n} A_i \times B_i}{\sqrt{\sum_{i=1}^{n} (A_i)^2} \times \sqrt{\sum_{i=1}^{n} (B_i)^2}}$$

**Patentansprüche**

1. Vorrichtung zur Vorhersage einer 3D-Form eines für einen Patienten maßgefertigten Implantats (100), umfassend:

  einen Speicher (120), der so konfiguriert ist, dass er wenigstens eine Anweisung zur Vorhersage der 3D-Form des für den Patienten maßangefertigten Implantats (100) speichern kann; und
  einen Prozessor (130), der so konfiguriert ist, dass er einen Arbeitsschritt gemäß der Anweisung ausführen kann, wobei der Prozessor (130) so konfiguriert ist, dass er:

    eine Korrelation zwischen einer 2D-Meniskusform und einer 3D-Meniskusform modellieren kann;
    die 3D-Meniskusform anhand von 3D-Formmerkmalen, die aus Modellierungsergebnissen abgeleitet sind, vorhersagen kann;
    2D-Meniskusformmerkmale anhand von 2D-Röntgen- und 2D-MRT-Bildern des gegenüberliegenden, gesunden Knies desselben Patienten messen kann, um die Korrelation zwischen der 2D-Meniskusform und der 3D-Meniskusform zu bestimmen;
    2D-Formmerkmale auswählen kann, die eine Korrelation zwischen den gemessenen 2D-Formmerkmalen und den 3D-Formmerkmalen oberhalb eines vorbestimmten Niveaus aufweisen, indem er eine Korrelationsanalyse durchführt;
    Werte für die 3D-Meniskusformmerkmale vorhersagen kann, indem er die ausgewählten 2D-Formmerkmale und demographische Merkmale in ein Meniskusform-Vorhersagemodell, das ein Regressions-CNN (neuronales Faltungsnetzwerk) umfasst, eingibt; und
    eine Meniskusgruppe erzeugt.

2. Vorrichtung gemäß Anspruch 1, wobei der Prozessor (130) so konfiguriert ist, dass er:

  eine Gruppierung durchführen kann, indem er eine Clusteranalyse auf der Basis des Mittelwerts und der Variation von 3D-Formen von normalen menschlichen Menisken durchführt, um aus vorab gespeicherten Meniskusdaten einen ähnlichen Meniskus herauszufinden; und
  unter den durch die Clusteranalyse erzeugten Clustern einen Cluster ermitteln und auswählen kann, der der vorhergesagten 3D-Meniskusform in Bezug auf Mittelwert und Standardabweichung der vorhergesagten 3D-Meniskusform am ähnlichsten ist.

3. Vorrichtung gemäß Anspruch 2, wobei der Prozessor (130) so konfiguriert ist, dass er:

ähnliche Merkmale unter Verwendung der Kosinus-Ähnlichkeit zwischen den vorhergesagten 3D-Meniskusformmerkmalen und Formmerkmalen von Trainingsdaten innerhalb des ausgewählten Clusters extrahieren kann; und

einen ähnlichsten Meniskus auswählen kann, indem er auf der Basis der extrahierten ähnlichen Merkmale einen ähnlichsten normalen menschlichen Meniskus erhält.

4. Vorrichtung gemäß Anspruch 1, wobei die 2D-Formmerkmale wenigstens eines oder mehrere aus der anteroposterioren (AP) Länge, mediolateralen (ML) Länge, dem Abstand zwischen Vorderhorn und Hinterhorn (AH-PH), der Länge des medialen Femurkondylus (Med FC), Länge des lateralen Femurkondylus (Lat FC) und dem Gelenkspalt in einer Stress-Röntgenaufnahme umfassen.

5. Vorrichtung gemäß Anspruch 1, wobei die 3D-Formmerkmale wenigstens eines oder mehrere aus der anteroposterioren (AP) Länge, mediolateralen (ML) Länge, dem Abstand zwischen Vorderhorn und Hinterhorn (AH-PH), innerem Umfang, äußerem Umfang, Abdeckungsfläche und Spaltfläche umfassen.

6. Vorrichtung gemäß Anspruch 2, wobei der Prozessor (130) so konfiguriert ist, dass er:

eine k-Means-Clusteranalyse in k Gruppen unter Verwendung von gemessenen 3D-Formparametern durchführen kann; und

einen Mittelwert zwischen 3D-Formparametern innerhalb jedes Clusters messen kann.

7. Vorrichtung gemäß Anspruch 3, wobei der Prozessor (130) so konfiguriert ist, dass er die Kosinus-Ähnlichkeit unter Verwendung eines Merkmalsvektors des vorhergesagten 3D-Meniskus und eines Merkmalsvektors eines Mittelwert-Clusters berechnen kann.

8. Vorrichtung gemäß Anspruch 7, wobei die Kosinus-Ähnlichkeit durch die folgende Gleichung 1 berechnet wird, wobei $A_i$ ein i-ter Merkmalsvektor des vorhergesagten 3D-Meniskus ist und $B_i$ der Merkmalsvektor des Mittelwert-Clusters ist:

[Gleichung 1]

$$\text{Ähnlichkeit} = \cos(\theta) = \frac{A \cdot B}{\|A\|\|B\|} = \frac{\sum_{i=1}^{n} A_i \times B_i}{\sqrt{\sum_{i=1}^{n}(A_i)^2} \times \sqrt{\sum_{i=1}^{n}(B_i)^2}}$$

9. Verfahren zur Vorhersage einer 3D-Form eines für einen Patienten maßgefertigten Implantats (100), wobei das Verfahren durch einen Prozessor (130) einer Vorrichtung durchgeführt wird, umfassend:

das Modellieren einer Korrelation zwischen einer 2D-Meniskusform und einer 3D-Meniskusform;

das Vorhersagen der 3D-Meniskusform anhand von 3D-Formmerkmalen, die aus Modellierungsergebnissen abgeleitet sind;

das Messen von 2D-Meniskusformmerkmalen anhand von 2D-Röntgen- und 2D-MRT-Bildern des gegenüberliegenden, gesunden Knies desselben Patienten, um die Korrelation zwischen der 2D-Meniskusform und der 3D-Meniskusform zu bestimmen;

das Auswählen von 2D-Formmerkmalen, die eine Korrelation zwischen den gemessenen 2D-Formmerkmalen und den 3D-Formmerkmalen oberhalb eines vorbestimmten Niveaus aufweisen, indem man eine Korrelationsanalyse durchführt;

das Vorhersagen von Werten für die 3D-Meniskusformmerkmale, indem man die ausgewählten 2D-Formmerkmale und demographische Merkmale in ein Meniskusform-Vorhersagemodell, das ein Regressions-CNN umfasst, eingibt; und

das Erzeugen einer Meniskusgruppe.

10. Verfahren gemäß Anspruch 9, weiterhin umfassend:

das Durchführen einer Gruppierung, indem man eine Clusteranalyse auf der Basis des Mittelwerts und der Variation von 3D-Formen von normalen menschlichen Menisken durchführt, um aus vorab gespeicherten Meniskusdaten einen ähnlichen Meniskus herauszufinden; und

das Ermitteln und Auswählen eines Clusters, der der vorhergesagten 3D-Meniskusform in Bezug auf Mittelwert und Standardabweichung der vorhergesagten 3D-Meniskusform am ähnlichsten ist, unter den durch die Clusteranalyse erzeugten Clustern.

11. Verfahren gemäß Anspruch 10, weiterhin umfassend:

das Extrahieren ähnlicher Merkmale unter Verwendung der Kosinus-Ähnlichkeit zwischen den vorhergesagten 3D-Meniskusformmerkmalen und Formmerkmalen von Trainingsdaten innerhalb des ausgewählten Clusters; und

das Auswählen eines ähnlichsten Meniskus, indem man auf der Basis der extrahierten ähnlichen Merkmale einen ähnlichsten normalen menschlichen Meniskus erhält.

12. Verfahren gemäß Anspruch 9, wobei die 2D-Formmerkmale wenigstens eines oder mehrere aus der anteroposterioren (AP) Länge, mediolateralen (ML) Länge, dem Abstand zwischen Vorderhorn und Hinterhorn (AH-PH), der Länge des medialen Femurkondylus (Med FC), Länge des lateralen Femurkondylus (Lat FC) und dem Gelenkspalt in einer Stress-Röntgenaufnahme umfassen.

13. Verfahren gemäß Anspruch 9, wobei die 3D-Formmerkmale wenigstens eines oder mehrere aus der anteroposterioren (AP) Länge, mediolateralen (ML) Länge, dem Abstand zwischen Vorderhorn und Hinterhorn (AH-PH), innerem Umfang, äußerem Umfang, Abdeckungsfläche und Spaltfläche umfassen.

14. Verfahren gemäß Anspruch 10, weiterhin umfassend:

das Durchführen einer k-Means-Clusteranalyse in k Gruppen unter Verwendung von gemessenen 3D-Formparametern; und

das Messen eines Mittelwerts zwischen 3D-Formparametern innerhalb jedes Clusters.

15. Verfahren gemäß Anspruch 11, weiterhin umfassend:
das Berechnen der Kosinus-Ähnlichkeit unter Verwendung eines Merkmalsvektors des vorhergesagten 3D-Meniskus und eines Merkmalsvektors eines Mittelwert-Clusters.

16. Verfahren gemäß Anspruch 15, wobei die Kosinus-Ähnlichkeit durch die folgende Gleichung 1 berechnet wird, wobei $A_i$ ein i-ter Merkmalsvektor des vorhergesagten 3D-Meniskus ist und $B_i$ der Merkmalsvektor des Mittelwert-Clusters ist:

[Gleichung 1]

$$\text{Ähnlichkeit} = \cos(\theta) = \frac{A \cdot B}{\|A\|\|B\|} = \frac{\sum_{i=1}^{n} A_i \times B_i}{\sqrt{\sum_{i=1}^{n}(A_i)^2} \times \sqrt{\sum_{i=1}^{n}(B_i)^2}}$$

**Revendications**

1. Dispositif pour prédire la forme 3D d'un implant sur mesure pour un patient (100), comprenant :

une mémoire (120) configurée pour stocker au moins une instruction permettant de prédire la forme 3D de l'implant sur mesure pour le patient (100) ; et
un processeur (130) configuré pour exécuter une opération selon l'instruction,
dans lequel le processeur (130) est configuré pour :

modéliser une corrélation entre la forme d'un ménisque en 2D et la forme d'un ménisque en 3D ;

prédire la forme 3D du ménisque à partir des caractéristiques de forme 3D dérivées des résultats de modélisation ;

mesurer les caractéristiques de forme du ménisque 2D à partir d'images radiographiques 2D et d'images IRM 2D d'un genou sain opposé du même patient afin de déterminer la corrélation entre la forme du ménisque 2D et la forme du ménisque 3D ;

sélectionner les caractéristiques de forme 2D qui présentent une corrélation entre les caractéristiques de forme 2D mesurées et les caractéristiques de forme 3D au-dessus d'un niveau prédéterminé en effectuant une analyse de corrélation ;

prédire les valeurs des caractéristiques de forme 3D du ménisque en introduisant les caractéristiques de forme 2D sélectionnées et les caractéristiques démographiques dans un modèle de prédiction de la forme du ménisque, qui comprend un réseau de neurones convolutif de régression ; et

générer un groupe ménisques.

2. Dispositif selon la revendication 1, dans lequel le processeur (130) est configuré pour :

effectuer un regroupement en effectuant un clustering basé sur la moyenne et la variation des formes 3D des ménisques humains normaux afin de trouver un ménisque similaire à partir de données de ménisques pré-enregistrées ; et

détecter et sélectionner le cluster le plus similaire à la forme 3D prédite du ménisque en termes de moyenne et d'écart type de la forme 3D prédite du ménisque parmi les clusters générés par le clustering.

3. Dispositif selon la revendication 2, dans lequel le processeur (130) est configuré pour :

extraire des caractéristiques similaires en utilisant la similarité cosinus entre les caractéristiques de forme du ménisque 3D prédites et les caractéristiques de forme des données d'entraînement au sein du cluster sélectionné ; et

sélectionner un ménisque le plus similaire en obtenant un ménisque humain normal le plus similaire sur la base des caractéristiques similaires extraites.

4. Dispositif selon la revendication 1, dans lequel les caractéristiques de forme 2D comprennent au moins l'une ou plusieurs parmi la longueur antéropostérieure (AP), la longueur médio-latérale (ML), la distance corne antérieure-corne postérieure (AH-PH), la longueur du condyle fémoral médial (Med FC), la longueur du condyle fémoral latéral (Lat FC) et l'espace articulaire sur la radiographie sous contrainte.

5. Dispositif selon la revendication 1, dans lequel les caractéristiques de forme 3D comprennent au moins l'une ou plusieurs parmi la longueur antéropostérieure (AP), la longueur médio-latérale (ML), la distance corne antérieure-corne postérieure (AH-PH), la circonférence intérieure, la circonférence extérieure, la zone de couverture et la zone d'espace.

6. Dispositif selon la revendication 2, dans lequel le processeur (130) est configuré pour :

effectuer un clustering K-means en K groupes à l'aide des paramètres de forme 3D mesurés ; et

calculer une moyenne des paramètres de forme 3D au sein de chaque cluster.

7. Dispositif selon la revendication 3, dans lequel le processeur (130) est configuré pour : calculer la similarité cosinus en utilisant un vecteur de caractéristiques du ménisque 3D prédit et un vecteur de caractéristiques d'un cluster moyen.

8. Dispositif selon la revendication 7, dans lequel la similarité cosinus est calculée à l'aide de l'équation 1 suivante, où $A_i$ est un i-ème vecteur de caractéristiques du ménisque 3D prédit, et $B_i$ est le vecteur de caractéristiques du cluster moyen :

[équation 1]

$$\text{Similarité} = \cos(\theta) = \frac{A \cdot B}{\|A\|\|B\|} = \frac{\sum\limits_{i=1}^{n} A_i \times B_i}{\sqrt{\sum\limits_{i=1}^{n} (A_i)^2} \times \sqrt{\sum\limits_{i=1}^{n} (B_i)^2}}.$$

9. Méthode pour prédire la forme 3D d'un implant sur mesure pour un patient (100), la méthode étant mise en œuvre par un processeur (130) d'un dispositif, comprenant les étapes consistant à :

modéliser une corrélation entre la forme d'un ménisque en 2D et la forme d'un ménisque en 3D ;
prédire la forme 3D du ménisque à partir des caractéristiques de forme 3D dérivées des résultats de modélisation ;
mesurer les caractéristiques de forme du ménisque 2D à partir d'images radiographiques 2D et d'images IRM 2D d'un genou sain opposé du même patient afin de déterminer la corrélation entre la forme du ménisque 2D et la forme du ménisque 3D ;
sélectionner les caractéristiques de forme 2D qui présentent une corrélation entre les caractéristiques de forme 2D mesurées et les caractéristiques de forme 3D au-dessus d'un niveau prédéterminé en effectuant une analyse de corrélation ;
prédire les valeurs des caractéristiques de forme 3D du ménisque en introduisant les caractéristiques de forme 2D sélectionnées et les caractéristiques démographiques dans un modèle de prédiction de la forme du ménisque, qui comprend un réseau de neurones convolutif de régression ; et
générer un groupe ménisques.

10. Méthode selon la revendication 9, comprenant en outre les étapes consistant à :

effectuer un regroupement en effectuant un clustering basé sur la moyenne et la variation des formes 3D des ménisques humains normaux afin de trouver un ménisque similaire à partir de données de ménisques pré-enregistrées ; et
détecter et sélectionner le cluster le plus similaire à la forme 3D prédite du ménisque en termes de moyenne et d'écart type de la forme 3D prédite du ménisque parmi les clusters générés par le clustering.

11. Méthode selon la revendication 10, comprenant en outre les étapes consistant à :

extraire des caractéristiques similaires en utilisant la similarité cosinus entre les caractéristiques de forme du ménisque 3D prédites et les caractéristiques de forme des données d'entraînement au sein du cluster sélectionné ; et
sélectionner un ménisque le plus similaire en obtenant un ménisque humain normal le plus similaire sur la base des caractéristiques similaires extraites.

12. Méthode selon la revendication 9, dans laquelle les caractéristiques de forme 2D comprennent au moins l'une ou plusieurs parmi la longueur antéropostérieure (AP), la longueur médio-latérale (ML), la distance corne antérieure-corne postérieure (AH-PH), la longueur du condyle fémoral médial (Med FC), la longueur du condyle fémoral latéral (Lat FC) et l'espace articulaire sur la radiographie sous contrainte.

13. Méthode selon la revendication 9, dans laquelle les caractéristiques de forme 3D comprennent au moins l'une ou plusieurs parmi la longueur antéropostérieure (AP), la longueur médio-latérale (ML), la distance corne antérieure-corne postérieure (AH-PH), la circonférence intérieure, la circonférence extérieure, la zone de couverture et la zone d'espace.

14. Méthode selon la revendication 10, comprenant en outre les étapes consistant à :

effectuer un clustering K-means en K groupes à l'aide des paramètres de forme 3D mesurés ; et
calculer une moyenne des paramètres de forme 3D au sein de chaque cluster.

15. Méthode selon la revendication 11, comprenant en outre l'étape consistant à :
calculer la similarité cosinus en utilisant un vecteur de caractéristiques du ménisque 3D prédit et un vecteur de caractéristiques d'un cluster moyen.

16. Méthode selon la revendication 15, dans laquelle la similarité cosinus est calculée à l'aide de l'équation 1 suivante, où $A_i$ est un i-ème vecteur de caractéristiques du ménisque 3D prédit, et $B_i$ est le vecteur de caractéristiques du cluster moyen :

[équation 1]

$$\text{Similarité} = \cos(\theta) = \frac{A \cdot B}{\|A\|\|B\|} = \frac{\sum_{i=1}^{n} A_i \times B_i}{\sqrt{\sum_{i=1}^{n} (A_i)^2} \times \sqrt{\sum_{i=1}^{n} (B_i)^2}}.$$

FIG. 1

FIG. 2

FIG. 3

FIG. 4

(a)                                                                    (b)

Input

2D shape feature
· AP length
· ML length
· AH-PH distance
· Med FC condylar length
· Lat FC condylar length
· Joing space in stress X-ray

Demographics
· Height

Input | Conv | BN | ReLU | AP | CCN | MPConv | MP | Drop | FC | Regression (shape feature)

1x5 | 1x20 | · · · | 1x20 | 1xNF$_k$ · · · 1xNF$_k$ | 1x8 | 1x8

Feature Extraction

Prediction

Output

Predicted 3D Shape feature
· AP length
· ML length
· AH-PH distance
· Inner Circumference
· Outer Circumference
· Coverage Area
· Gap Area

FIG. 5

FIG. 6

K-means clustering

FIG. 7

Start

Extract and select meniscus features — S100

Predict meniscus shape features — S200

Select cluster — S300

Select meniscus based on cosine similarity — S400

End

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8965088 B2

- KR 102228087 B1

**Non-patent literature cited in the description**

- **VRANCKEN A. C. et al.** *3D geometry analysis of the medial meniscus - a statistical shape modeling approach*
- **LEWIS KIRA et al.** *Calculation of the Meniscus Shape Formed under Gravitational Force by Solving the Young-Laplace Differential Equation Using the Bézier Curve Method*

- **A. TACK et al.** *Knee menisci segmentation using convolutional neural networks: data from the Osteoarthritis Initiative*
- **VASWANI.** *Attention Is All You Need*, 2017